**Europäisches Patentamt**

**European Patent Office**

**Office européen des brevets**

(19)

(11) Veröffentlichungsnummer : **0 412 337 B1**

(12) # EUROPÄISCHE PATENTSCHRIFT

(45) Veröffentlichungstag der Patentschrift :
**01.09.93 Patentblatt 93/35**

(51) Int. Cl.$^5$ : **C07C 31/20,** C07C 29/141, C07C 29/04

(21) Anmeldenummer : **90113908.9**

(22) Anmeldetag : **20.07.90**

(54) **Verfahren zur Herstellung von 1,3-Propandiol.**

(30) Priorität : **08.08.89 DE 3926136**

(43) Veröffentlichungstag der Anmeldung :
**13.02.91 Patentblatt 91/07**

(45) Bekanntmachung des Hinweises auf die Patenterteilung :
**01.09.93 Patentblatt 93/35**

(84) Benannte Vertragsstaaten :
**AT BE CH DE DK ES FR GB IT LI LU NL SE**

(56) Entgegenhaltungen :
**AT-B- 313 247
DE-A- 2 604 095
US-A- 3 536 763**

(73) Patentinhaber : **Degussa Aktiengesellschaft
Weissfrauenstrasse 9
D-60311 Frankfurt (DE)**

(72) Erfinder : **Arntz, Dietrich, Dr.
Lorsbachstrasse 32
D-6370 Oberursel (DE)**
Erfinder : **Wiegand, Norbert, Dr.
Birkenhainer Strasse 15a
D-6450 Hanau 1 (DE)**

**Beschreibung**

Die Erfindung betrifft ein Verfahren zur Herstellung von 1,3-Propandiol durch Hydratisierung von Acrolein in Gegenwart eines sauren Kationenaustauschers mit nachfolgender katalytischer Hydrierung des 3-Hydroxypropionaldehyds.

1,3-Propandiol besitzt als Monomerbaustein für Polyester und Polyurethane sowie als Ausgangsstoff für die Synthese cyclischer Verbindungen vielseitige Anwendungsmöglichkeiten. Zur Herstellung von 1,3-Propandiol sind schon viele Verfahren vorgeschlagen worden, darunter solche, welche einen Molekülaufbau aus einem $C_2$- und $C_1$-Baustein umfassen und solche, welche von einem $C_3$-Baustein, wie insbesondere Acrolein, ausgehen.

Wie aus der US-PS 2,434,110 bekannt ist, läßt sich Acrolein in Gegenwart eines sauren Katalysators hydratisieren, wobei 3-Hydroxypropionaldehyd gebildet wird. Die Umsetzung erfolgt vorzugsweise bei erhöhter Temperatur unter Verwendung einer 5 bis 30 gew.-%igen Lösung von Acrolein in Wasser und einer Säure, wie z. B. Schwefelsäure, Phosphorsäure oder sauren Salzen dieser Säuren, als Katalysator. Das bei der Hydratisierung erhaltene Reaktionsgemisch wird, gegebenenfalls nach Entfernung von nicht umgesetztem Acrolein, in Gegenwart üblicher Hydrierkatalysatoren hydriert. Für die Hydrierung des 3-Hydroxypropionaldehyds zu 1,3-Propandiol sind Katalysatoren, welche ein oder mehrere hydrierwirksame Metalle, wie z. B. Fe, Co, Ni, Cu, Ag, Mo, W, V, Cr, Rh, Pd, Os, Ir, Pt enthalten, geeignet.

Nachteilig an dem Verfahren der US-PS 2,434,110 sind die niedrigen Ausbeuten an 1,3-Propandiol, welche insbesondere auf Acrolein verbrauchende Kondensationsreaktionen während der Hydratisierungsstufe zurückzuführen sind. Die Selektivität der mineralsauer katalysierten Hydratisierung ist ferner außergewöhnlich stark vom Acrolein-Umsatz abhängig; um eine akzeptable Selektivität zu erzielen, beendet man die Hydratisierung bei niedrigem Acrolein-Umsatz, was aber gleichzeitig eine geringe Raum-Zeit-Ausbeute nach sich zieht.

Es hat nicht an Versuchen gefehlt, die Nachteile des zuvor gewürdigten Verfahrens zu mindern, beispielsweise durch Anlagerung von niederen Carbonsäuren an die Doppelbindung des Acroleins, was aber eine Verseifung im Anschluß an die Hydrierung erforderlich macht; zusätzlich bereitet die Recyclierung der Carbonsäure Schwierigkeiten (US 2,638,479). Bekannt ist auch die Hydratisierung von Acrolein mit Kohlendioxid als Katalysator, jedoch erfordert dieses Verfahren lange Reaktionszeiten - vgl. DE-OS 19 05 823.

Es wurde festgestellt, daß unter Verwendung von z. B. Phosphorsäure oder Dihydrogenphosphaten als Katalysator zwar die Hydratisierung von Acrolein durchgeführt werden kann, bei der anschließenden Hydrierung aber Probleme auftreten, wenn man das vom nicht umgesetzten Acrolein befreite Reaktionsgemisch einsetzt.

Bei Verwendung von an sich sehr wirksamen Nickel-Hydrierkatalysatoren sinken bei wiederholtem Einsatz des Katalysators sowohl der Hydrierumsatz als auch die Reaktionsgeschwindigkeit rasch ab. Dies führt zu einem erhöhten Katalysatorverbrauch.

Zusätzlich kommt es durch die Anwesenheit des Hydratisierungskatalysators während der destillativen Aufarbeitung zu Produktverlusten durch Zersetzung bzw. im Falle einer vorangehenden Neutralisation zu Verstopfungen und Verkrustungen in der Anlage. Die aufgezeigten Probleme lassen sich zum Teil vermindern, wenn man den Hydratisierungskatalysator mittels Ionenaustauscherharzen vor der Hydrierung aus dem Reaktionsgemisch entfernt oder 3-Hydroxypropionaldehyd extraktiv aus dem Reaktionsgemisch abtrennt und dann hydriert. Beide alternativen Maßnahmen zur Reduzierung des Verbrauchs an teurem Hydrierkatalysator machen aber zusätzliche Einrichtungen erforderlich, führen zu einem höheren Energieaufwand und zusätzlich zu Abwasserproblemen und erhöhen somit die Herstellkosten für 1,3-Propandiol.

Gemäß dem Verfahren der US-PS 3,536,763 wird die Hydratisierung in Gegenwart schwachsaurer Kationenaustauscherharze, deren funktionelle Gruppen Carboxylgruppen sind, bei 40 bis 120 °C durchgeführt. Vorzugsweise sollen 0,1 bis 5 % der funktionellen Gruppen in Form eines Alkali-, Erdalkali- oder Erdmetallcarboxylats vorliegen. Die Ausbeuten an 3-Hydroxypropionaldehyd werden mit etwa 80 % angegeben, wobei die Ausbeuten vom Acrolein-Umsatz im Bereich von 25 bis 65 % praktisch nicht abhängig sein sollen. Dieses Dokument umfaßt auch die an sich bekannte Hydrierung des 3-Hydroxypropionaldehyds zu 1,3-Propandiol.

Bei der Nacharbeitung des Verfahrens der US-PS 3,536,763 konnte zwar die katalytische Wirksamkeit der Ionenaustauscherharze mit Carboxylgruppen bestätigt werden, der Grad der Wirksamkeit ließ aber die Verwendung dieser Ionenaustauscher in technische Anlagen als nicht geeignet erscheinen. Es zeigte sich nämlich, daß diese Katalyatoren höhere Temperaturen und längere Reaktionszeiten erfordern, was der erwünschten hohen Raum-Zeit-Ausbeute zuwiderläuft.

Aufgabe der vorliegenden Erfindung ist somit, ein verbessertes Verfahren zur Herstellung von 1,3-Propandiol durch Hydratisierung von Acrolein in Gegenwart von sauren Kationenaustauscherharzen mit nachfolgender katalytischer Hydrierung zur Verfügung zu stellen, das mit höherer Raum-Zeit-Ausbeute und guter Selektivität in der Hydratisierungsstufe durchgeführt werden kann. Das bei der Hydratisierung erhaltene Re-

2

aktionsgemisch sollte auch die Aktivität des Hydrierkatalysators möglichst wenig beeinträchtigen, um die Wiederverwendung des Katalysators in Folgechargen zu ermöglichen und damit die Wirtschaftlichkeit des Verfahrens zu verbessern.

Das erfindungsgemäße Verfahren zur Herstellung von 1,3-Propandiol durch Hydratisierung von Acrolein in Gegenwart eines sauren Kationenaustauscherharzes unter Bildung von 3-Hydroxypropionaldehyd, wobei Acrolein und Wasser im Gewichtsverhältnis von 1:2 bis 1:20 bei 30 bis 120 °C und einem Druck im Bereich von 1 bis 20 bar umgesetzt werden, Abtrennung des Ionenaustauschers und, soweit vorhanden, des nicht umgesetzten Acroleins aus dem Reaktionsgemisch und anschließende katalytische Hydrierung des 3-Hydroxypropionaldehyds unter an sich bekannten Bedingungen in flüssiger oder gasförmiger Phase unter Verwendung üblicher Hydrierkatalysatoren, ist dadurch gekennzeichnet, daß man Kationenaustauscherharze mit Phosphonsäuregruppen verwendet.

Die erfindungsgemäß einzusetzenden Kationenaustauscherharze enthalten saure Phosphonsäuregruppen. Die Phosphonsäuregruppen können zum Teil auch als saure Salze von Alkali- oder Erdalkalimetallen vorliegen, bevorzugt sind aber im allgemeinen die freien Phosphonsäuregruppen. Die Ionenaustauscher, welche im Reaktionsmedium unlöslich sind, basieren auf einer Polymermatrix mit Vernetzungsstellen und enthalten die für die katalytische Wirksamkeit verantwortlichen Phosphonsäuregruppen chemisch eingebaut. Die Phosphonsäuregruppe ist bevorzugt an ein aliphatisches C-Atom gebunden. Besonders geeignet sind Ionenaustauscher mit chelatbildenden Aminophosphonsäuregruppen, in welchen eine Aminogruppe über ein oder zwei C-Atome mit der Phosphonsäuregruppe verbunden ist. Herausragende Eigenschaften im erfindungsgemäßen Verfahren haben Austauscherharze mit einer Polystyrolmatrix, welche mit Divinylbenzol vernetzt ist und als funktionelle Gruppe Aminomethanphosphonsäuregruppen der Struktur $-NH-CH_2-PO_3H_2$ enthält. Die Dichte der funktionellen Gruppen liegt im allgemeinen im Bereich um 1 bis 3 Äquivalente ($H^+$-Form) pro 1 Austauscherharz. Das Austauscherharz wird bevorzugt in der für Ionenaustauscher üblichen Perlform eingesetzt.

Acrolein und Wasser werden in einem Gewichtsverhältnis von 1:2 bis 1:20, insbesondere 1:3 bis 1:10 und bevorzugt 1:3 bis 1:6, der Hydratisierungsstufe zugeführt. Die Umsetzung zum 3-Hydroxypropionaldehyd erfolgt im Temperaturbereich von 30 bis 120 °C. Eine Temperatur im Bereich von 40 °C oder über 100 °C wird bevorzugt; eine Temperatur unter 40 °C führt im allgemeinen zu langen Reaktionszeiten, eine Temperatur oberhalb 100 °C zu einer verminderten Selektivität und Problemen bezüglich der Standzeit der Austauscherharze. Besonders bevorzugt erfolgt die Hydratisierung bei 50 bis 90 °C.

Im Temperaturbereich unter dem Siedepunkt des Acroleins kann die Umsetzung bei Normaldruck oder mäßigem Druck erfolgen. Bei Umsetzungstemperaturen um oder über dem Siedepunkt von Acrolein wird man unter einem Druck im Bereich von etwa 2 bis 20 bar arbeiten. Im besonders bevorzugten Temperaturbereich von 50 bis 90 °C hat sich ein Druck im Bereich von 2 bis 5 bar als geeignet erwiesen.

Üblicherweise nimmt die Selektivität der Umsetzung von Wasser mit Acrolein zu 3-Hydroxypropionaldehyd mit steigendem Acrolein-Umsatz ab - eine vergleichbare Selektivitätsabnahme wurde auch bei den Kationenaustauschern mit Carboxylgruppen beobachtet. Durch Erniedrigung des Acrolein-Umsatzes von etwa 80 % auf etwa 30 % steigt die Ausbeute an 1,3-Propandiol, wenn das Gemisch aus der Hydratisierung nach Abdestillieren des nicht-umgesetzten Acroleins mit Raney-Nickel hydriert wird, von etwa 60 % auf über 80 %, bezogen auf den Acrolein-Umsatz. Ein Acrolein-Umsatz zwischen 30 und 80 %, insbesondere 40 bis 60 % wird daher bevorzugt.

Sofern erwünscht, können dem Acrolein-Wasser-Gemisch Polymerisationsinhibitoren, wie z. B. Hydrochinon, Hydrochinonmonomethylether oder butylierte Phenole, in wirksamer Menge zugegeben werden.

Die Hydratisierung kann diskontinuierlich und kontinuierlich erfolgen, wobei an sich bekannte Reaktoren, wie beispielsweise Rührreaktoren, Schlaufenreaktoren, Schwebebett-, Wirbelbett- und Strömungsrohrreaktoren, einsetzbar sind. Nach dem Strömungsrohrprinzip arbeitende Reaktoren werden gegenüber Schlaufen- und Rührreaktoren bevorzugt. Die Durchflußgeschwindigkeit durch ein Strömungsrohr, das den Phosphonsäuregruppen aufweisenden sauren Kationenaustauscher enthält und mit einem heizbaren Mantel versehen ist, sowie die Reaktionstemperatur wird man so einstellen, daß der gewünschte Acrolein-Umsatz erreicht wird. Die Reaktionsmischung kann auch mehrfach über das Ionenaustauscherbett geführt werden, sofern größere Totvolumina vermieden werden, in welchen die Reaktionsmischung bei Reaktionstemperatur in Abwesenheit des Hydratisierungskatalysators vorliegt.

Nach der Abtrennung des Ionenaustauschers, was üblicherweise durch Sedimentation oder Filtration erfolgt oder sich bei Verwendung von an sich bekannten Einrichtungen für den Ionenaustausch praktisch von allein ergibt, wird das Reaktionsgemisch, soweit erforderlich, von nicht-umgesetztem Acrolein befreit. Bei einem Acrolein-Umsatz zwischen 30 bis 80 % wird diese Maßnahme stets empfehlenswert sein, weil Acrolein, auch wenn dieses als wasserhaltiges Acrolein abgetrennt worden ist, direkt in den Prozeß zurückgeführt werden kann. Die Abtrennung des Acroleins kann in bekannter Weise, insbesondere destillativ, vorzugsweise unter vermindertem Druck und Temperaturen unter 80 °C, verwirklicht werden. Besonders günstig ist es, das Reaktionsgemisch einem Dünnschichtverdampfer zuzuführen, wobei unter schonenden Bedingungen sowohl nicht-umgesetztes Acrolein als auch ein Teil des Wassers des Reaktionsgemisches gemeinsam abdestilliert werden. Auf diese Weise erhält man als Sumpfprodukt eine konzentrierte wäßrige Lösung von 3-Hydroxypropionaldehyd, was sich vorteilhaft auf die Hydrierung und den Energieverbrauch des Verfahrens auswirkt.

Sofern dies erwünscht wird, kann aus dem vom Acrolein befreiten wäßrigen Reaktionsgemisch der 3-Hydroxypropionaldehyd mittels polarer organischer Lösungsmittel, wie z. B. einem niederen Ester oder Isobutanol, extrahiert und in dieser Form der Hydrierung zugeführt werden. Diese Ausführungsform führt in der Hydrierstufe zwar zu einer raschen $H_2$-Aufnahme, dem stehen jedoch der nicht unbeträchtliche technische Aufwand für die Extraktion und Rückgewinnung des organischen Lösungsmittels entgegen. Somit wird die direkte Hydrierung des wäßrigen 3-Hydroxypropionaldehyds bevorzugt.

Die katalytische Hydrierung des 3-Hydroxypropionaldehyds in flüssiger Phase wird in an sich bekannter Weise und in üblichen Hydrierapparaturen durchgeführt. Der Katalysator kann sowohl in suspendierter Form per se oder trägergebunden vorliegen oder Bestandteil von Festbettkatalysatoren sein; auch homogene Katalysatoren können herangezogen werden. Als Suspensionskatalysatoren sind Raney-Nickel, das mit verschiedenen anderen katalytisch wirksamen Metallen dotiert sein kann, sowie Platin auf Aktivkohle besonders geeignet. Unter den Festbettkatalysatoren kommen die bei der Würdigung der US-PS 2,434,110 genannten Stoffe zur Anwendung; Nickelkatalysatoren haben sich als besonders wirkungsvolle Katalysatoren erwiesen. Eine hohe Raum-Zeit-Ausbeute wird in der Hydrierstufe erzielt, wenn die zu hydrierende Lösung einen pH-Wert im Bereich von 2,5 bis 6,5, vorzugsweise um 6, aufweist und die Hydriertemperatur im Bereich zwischen 25 und 100 °C liegt.

Prinzipiell kann 3-Hydroxypropionaldehyd auch in der Gasphase katalytisch hydriert werden - vgl. DE-PS 20 54 601, so daß sich an die erfindungsgemäße Hydratisierung auch diese Ausführungsform der Hydrierung anschließen kann.

Wie aus den Beispielen folgt, erlauben die erfindungsgemäß zu verwendenden Kationenaustauscher mit Phosphonsäuregruppen eine wesentlich höhere Raum-Zeit-Ausbeute zu erzielen, als dies mit den vorbekannten Ionenaustauschern mit Carboxylgruppen der Fall war. Die vorteilhafte Wirkung der Phosphonsäuregruppen-haltigen Ionenaustauscherharze ist dabei Folge der höheren Reaktivität; gleichzeitig kann die Reaktionstemperatur niedriger gehalten und damit eine höhere Selektivität erreicht werden. Während unter vergleichbaren Bedingungen in der gleichen Apparatur durchgeführte Versuche unter Verwendung der erfindungsgemäßen Austauscherharze LHSV-Werte (liquid hourly space velocity) um 0,6 Std.$^{-1}$ erhalten wurden, lagen die LHSV-Werte im Falle der Ionenaustauscher gemäß US-PS 3,536,763 nur um 0,4 Std.$^{-1}$.

Bei der Hydrierung von erfindungsgemäßen hergestellten wäßrigen 3-Hydroxypropanaldehyd-Lösungen werden Nickel-Hydrierkatalysatoren viel langsamer desaktiviert als im Falle von Lösungen, welche noch aus der Hydratisierung stammendes Phosphat enthalten. Im erfindungsgemäßen Verfahren läßt sich der Katalysator somit mehrfach wiederverwenden.

Beispiele 1 und 2

Hydratisierung von Acrolein unter Verwendung eines schwach sauren Ionenaustauschers (Lewatit CNP 80 der Firma Bayer) - Beispiel 1 / Stand der Technik - und eines sauren Ionenaustauschers mit Aminophosphonatgruppen (Duolite C 467 der Firma Rohm & Haas) - Beispiel 2 / erfindungsgemäß - in einer Umlaufapparatur bei 40 °C.

Die Umlaufapparatur umfaßt eine Doppelmantel-Glassäule (Länge 600 mm, innerer Durchmesser 25 mm), welche mit 300 ml Ionenaustauscherharz beschickt wird, eine Umwälzpumpe in der Umlaufleitung, ein 2 l Vorratsgefäß, Probenahmestutzen, pH-Elektrode und Thermometer sowie Rückflußkühler zwecks Druckausgleich.

Der Ionenaustauscher wird in bekannter Weise in die H-Form überführt; anschließend wird die gesamte Apparatur mit 1 l der wäßrigen Acroleinlösung gespült. Nach erneuter Beschickung der Apparatur mit 950 ml wäßriger Acroleinlösung, 17,8 gew.-%ig, wird diese Lösung über einen Zeitraum von mehreren Stunden bei 40 °C über das Ionenaustauscherharz gepumpt (2,7 l/h) und der Fortgang der Hydratisierung gaschromatographisch - Acrolein und 3-Hydroxypropionaldehyd (HPA) - und durch Aldehydtitration verfolgt.

Die erhaltenen Ergebnisse sind der nachfolgenden Tabelle zu entnehmen:

| Beispiel Nr. | Ionenaus-tauscher | Zeit h | pH | CHO-Konz. titr. %v.Eins. | Konzentration Ac. Gew.-% | HPA Gew.-% | Ac-Umsatz % | Umsatz-Ausbeute % |
|---|---|---|---|---|---|---|---|---|
| 1 | Lewatit CNP 80 | 0 | | 100 | 17,7 | – | – | – |
| | | 1 | 3,6 | 95,4 | 17,0 | – | 4,0 | – |
| | | 3 | 3,7 | 91,0 | 16,0 | 0,2 | 9,2 | 11 |
| | | 6 | 3,9 | 85,8 | 14,4 | 0,8 | 18,2 | 18 |
| 2 | Duolite C 467 | 0 | | 100 | 18,2 | – | – | – |
| | | 1 | 3,8 | 95,7 | 16,2 | 1,6 | 10,9 | 62 |
| | | 3 | 3,8 | 94,0 | 14,6 | 3,2 | 19,3 | 69 |
| | | 6 | 3,9 | 90,9 | 12,9 | 5,2 | 28,9 | 75 |
| | | 7 | 3,9 | 90,0 | 12,4 | 5,8 | 31,8 | 76 |

Ac      =   Acrolein

HPA    =   3-Hydroxypropionaldehyd

CHO-Konz.titr.  =  Aldehydkonzentration bestimmt durch Oximtitration

Ac und HPA wurden quantitativ gaschromatographisch bestimmt, wobei eine Mehrpunkteichung über einen inneren Standard unter Verwendung einer Porapak P-Säule erfolgte.

EP 0 412 337 B1

Der Ionenaustauscher mit Carboxylgruppen ist, wie aus dem zeitabhängigen Verlauf der Acrolein- und HPA-Konzentration folgt, viel weniger aktiv als der Ionenaustauscher mit erfindungsgemäßen Aminophosphatgruppen. Der Verlauf der durch Titration bestimmten Aldehydkonzentration weist ferner bei den erfindungsgemäß zu verwendenden Ionenaustauschern auf eine geringere Nebenproduktbildung hin.

Beispiele 3 bis 7

In einer Technikumsapparatur mit einem Schlaufenreaktor werden vorbekannte Kationenaustauscherharze mit Carboxylgruppen (Beispiele 3 und 4) und erfindungsgemäße Kationenaustauscherharze mit Aminophosphonatgruppen (Beispiele 5 bis 7) im kontinuierlichen Betrieb bei unterschiedlichen Temperaturen und Verweilzeiten miteinander verglichen, um die Raum-Zeit-Ausbeute - hier als LHSV-Wert angegeben - zu ermitteln.

Die Anlage besteht im wesentlichen aus Vorrichtungen zum Herstellen und Einspeisen der wäßrigen Acroleinlösung, der Reaktionsschlaufe, Vorlagen zur Aufnahme des Reaktionsproduktes sowie den Sicherheits-, Meß- und Regeleinrichtungen. Die Reaktionsschlaufe (6,2 l) besteht aus einem Doppelmantelrohr zur Thermostatisierung der Reaktionslösung, einer Umwälzpumpe und dem eigentlichen Reaktionsrohr, das das Katalysatorbett - 4 l Ionenaustauscherharz - enthält. Aus der Reaktionsschlaufe führt ein Abgang über ein Filter und Regelventil zu den Vorlagen.

Die Ionenaustauscherharze werden in bekannter Weise in die H-Form überführt und säurefrei gewaschen. Nach Befüllen des Reaktionsrohres mit dem Ionenaustauscher und Funktionsprüfung der Anlage werden Acrolein und Wasser im gewünschten Verhältnis in die Reaktionsschlaufe eingespeist und umgewälzt (ca. 190 l/h).

Nach Einstellung des Acrolein 3-Hydroxypropionaldehyd-Gleichgewichts, das durch gaschromatographische Untersuchung von Proben festgestellt wird, beginnt der eigentliche kontinuierliche Betrieb, wobei Temperatur, Druck, Volumenströme und Zusammensetzung erfaßt bzw. ermittelt werden. Der folgenden Tabelle sind die wesentlichen Betriebsparameter sowie die Analysenergebnisse zu entnehmen.

| Beispiel | Austauscher-harz | Acrolein-Konz. | Ac/$H_2O$-Einspei-sung | Reakt.-temp. | mittlere Verweil-zeit | LHSV | Acrolein Umsatz | HPA-Ausbeute bez. auf Ac-Umsatz |
|---|---|---|---|---|---|---|---|---|
| Nr. | | Gew.-% | l/h | °C | h | $h^{-1}$ | % | % |
| 3 | Lewatit CNP 80 | 19,3 | 1,09 | 80 | 5,69 | 1,27 | 65,0 | 61,0 |
| 4 | " | 21,7 | 1,61 | 80 | 3,84 | 0,40 | 53,9 | 67,0 |
| 5 | Duolite C 467 | 19,8 | 1,55 | 80 | 3,99 | 0,39 | 70,7 | 65,1 |
| 6 | " | 20,2 | 2,46 | 80 | 2,51 | 0,62 | 63,7 | 71,0 |
| 7 | " | 20,3 | 2,45 | 60 | 2,52 | 0,61 | 40,8 | 82,0 |

EP 0 412 337 B1

Der Vergleich der Beispiele 4 (Stand der Technik) mit 5 (erfindungsgemäß) zeigt, daß bei vergleichbaren Betriebsbedingungen bei Verwendung des erfindungsgemäßen Ionenaustauschers der Umsatz ohne Selektivitätseinbuße um ca. 15 % gegenüber jenem des Standes der Technik gesteigert werden kann. Zusätzlich gelingt es - vgl. Beispiel 6 - bei Verwendung des erfindungsgemäßen Ionenaustauschers, den LHSV-Wert um ca. 50 % zu erhöhen, wobei der Umsatz nur mäßig niedriger wird, gleichzeitig aber die Selektivität ansteigt; die Raum-Zeit-Ausbeute, also die produzierte Menge Hydroxypropionaldehyd pro Zeiteinheit und Reaktorvolumen, wird auf diese Weise um über 50 % erhöht (im Vergleich zu Beispiel 4). Wie sich aus Beispiel 7 im Vergleich zu Beispiel 4 ergibt, läßt sich erfindungsgemäß bei um 20 °C niedrigerer Reaktionstemperatur dennoch eine wesentlich höhere Raum-Zeit-Ausbeute erhalten, als dies nach dem Stand der Technik möglich war.

Beispiel 8

Hydrierung einer gemäß Beispiel 6 erhaltenen wäßrigen Lösung von 3-Hydroxypropionaldehyd (HPA) an einem Festbettkontakt (Ni/Al$_2$O$_3$/SiO$_2$).

Die Reaktionslösung des Beispiels 6 wird unter Verwendung eines Dünnschichtverdampfers bei etwa 65 °C und unter vermindertem Druck von nicht-umgesetztem Acrolein und gleichzeitig einem Teil des Wassers befreit. In den Hydrierreaktor üblicher Bauart werden 750 ml einer 1,49 Mol HPA enthaltenden wäßrigen Lösung eingebracht und über 140 g Katalysator umgewälzt. Bei etwa 55 °C wird bei einem H$_2$-Druck von 150 bar, der während der Hydrierung auf 106 bar absinkt, 4 Stunden hydriert. Der HPA-Umsatz beträgt 100 % die Ausbeute an 1,3-Propandiol 78 % (quantitative Gaschromatographie). Die Aufarbeitung der Reaktionslösung erfolgt in an sich bekannter Weise destillativ.

Beispiele 9 bis 11

In einer kontinuierlichen Laboranlage mit einem Strömungsrohrreaktor, der das erfindungsgemäße Kationenaustauscherharz mit Aminophosphonatgruppen enthält, wird bei einer ausgewählten Temperatur eine Acrolein-Wasser-Mischung zur Reaktion gebracht, die entstandene HPA-Lösung nach Abtrennung des unumgesetzten Acroleins katalytisch hydriert und das erhaltene 1,3-Propandiol bestimmt.

Die Anlage besteht im wesentlichen aus Vorrichtungen zum Herstellen und Einspeisen der wäßrigen Acroleinlösung, dem Reaktor, bestehend aus drei je 2500 mm langen, hintereinandergeschalteten thermostatierbaren Rohren aus Edelstahl, die mit insgesamt 11,6 l Ionenaustauscherharz gefüllt sind, sowie Filtern, Vorlagen und Meß- und Regeleinrichtungen.

**Hydrierung von 3-Hydroxypropionaldehyd (HPA) mit Hilfe eines Raney-Nickel-Typ-Katalysators**

Über einen 6stündigen Zeitraum wird eine 18,3 gew.-%ige Acroleinlösung bei 50 °C Reaktortemperatur und einer Katalysatorbelastung von LHSV = 0,25 h$^{-1}$ durch zwei der drei mit Ionenaustauscherharz gefüllten Rohre gepumpt. Der Acroleinumsatz beträgt 49,0 %. Die erhaltene Lösung wird bei schwachem Vakuum auf im Prinzip bekannte Weise von Acrolein befreit und portionsweise in einem Hochdruckautoklaven unter Verwendung von 11,6 g eines nickelhaltigen Suspensionskatalysators pro kg Lösung bei 50 °C und 135 bar Wasserstoff-Anfangsdruck unter mehrfacher Verwendung des Katalysators hydriert. Nach dem Filtrieren wird die gesamte Lösung am Rotationsverdampfer von der Hauptmenge Wasser befreit und aus dem Rückstand das 1,3-Propandiol durch Vakuumdestillation in 77,0 %iger Ausbeute, bezogen auf umgesetztes Acrolein, gewonnen.

**Hydrierung von 3-Hydroxypropionaldehyd (HPA) mit Hilfe eines Edelmetallkatalysators**

Eine durch Durchpumpen einer 18,6 gew.-%igen Acroleinlösung durch die beschriebenen drei Reaktorrohre bei 58 °C, bei einer Katalysatorbelastung von LHSV = 0,22 h$^{-1}$ und einem Acroleinumsatz von 69,1 %, entsprechend dem vorangegangenen Beispiel, gewonnene, von nicht umgesetztem Acrolein befreite HPA-Lösung, wird in gleicher Weise wie in Beispiel 9 beschrieben, hydriert, nur daß anstelle des nickelhaltigen Hydrierkatalysators ein pulverförmiger Platin-Aktivkohle-Katalysator mit einem Platingehalt von 10 % eingesetzt wird. In der eingeengten Lösung wird das erhaltene 1,3-Propandiol mittels Gaschromatographie bestimmt. Die Selektivität beträgt 74,5 %, bezogen auf umgesetztes Acrolein.

Eine auf prinzipiell gleiche Weise durch Durchpumpen einer 17,5 gew.-%igen Acroleinlösung durch eines der mit erfindungsgemäßen Ionenaustauscherharz gefüllten Reaktorrohre bei 58 °C, einer Katalysatorbelastung von LHSV = 0,69 h$^{-1}$ und einem Acroleinumsatz von 34,9 % gewonnene HPA-Lösung wird, wie im vorangegangenen Beispiel, nach Abtrennung des unumgesetzten Acroleins hydriert. Die durch quantitative Gaschromatographie bestimmte Selektivität, bezogen auf umgesetztes Acrolein, beträgt 85,1 %.

## Patentansprüche

1. Verfahren zur Herstellung von 1,3-Propandiol durch Hydratisierung von Acrolein in Gegenwart eines sauren Kationenaustauscherharzes unter Bildung von 3-Hydroxypropionaldehyd, wobei Acrolein und Wasser im Gewichtsverhältnis von 1:2 bis 1:20 bei 30 bis 120 °C und einem Druck im Bereich von 1 bis 20 bar umgesetzt werden, Abtrennung des Ionenaustauschers und, soweit vorhanden, des nicht umgesetzten Acroleins aus dem Reaktionsgemisch und anschließende katalytische Hydrierung des 3-Hydroxypropionaldehyds unter an sich bekannten Bedingungen in flüssiger oder gasförmiger Phase unter Verwendung üblicher Hydrierkatalysatoren,
dadurch gekennzeichnet,
daß man Kationenaustauscherharze mit Phosphonsäuregruppen verwendet.

2. Verfahren nach Anspruch 1,
dadurch gekennzeichnet,
daß man Kationenaustauscher mit chelatbildenden Aminophosphonsäuregruppen verwendet.

3. Verfahren nach Anspruch 1 oder 2,
dadurch gekennzeichnet,
daß man in der Hydratisierungsstufe Acrolein und Wasser im Gewichtsverhältnis von 1:3 bis 1:6 einsetzt.

4. Verfahren nach einem oder mehreren der Ansprüche 1 bis 3,
dadurch gekennzeichnet,
daß man die Hydratisierung bei 50 bis 90 °C durchführt.

5. Verfahren nach einem oder mehreren der Ansprüche 1 bis 4,
dadurch gekennzeichnet,
daß man die Hydratisierung bis zu einem Acrolein-Umsatz von 30 bis 80 % betreibt, anschließend den Ionenaustauscher abtrennt und dann nicht-umgesetztes Acrolein zwecks Rückführung in die Hydratisierungsstufe aus dem wäßrigen Reaktionsgemisch abdestilliert.

6. Verfahren nach einem oder mehreren der Ansprüche 1 bis 5,
dadurch gekennzeichnet,
daß man die Hydratisierung in einem Strömungsrohr durchführt, wobei der gewünschte Acrolein-Umsatz durch einmalige Passage des Reaktionsgemisches durch das Strömungsrohr erreicht wird.

7. Verfahren nach einem oder mehreren der Ansprüche 1 bis 6,
dadurch gekennzeichnet,
daß man die katalytische Hydrierung des 3-Hydroxypropionaldehyds in wäßriger Lösung bei einem pH-Wert im Bereich von 2,5 bis 6,5 und einer Temperatur von 50 bis 90 °C durchführt.

8. Verfahren nach einem oder mehreren der Ansprüche 1 bis 7,
dadurch gekennzeichnet,
daß man 3-Hydroxypropionaldehyd in Gegenwart von nickelhaltigen Suspensions- oder Festbettkatalysatoren durchführt.

## Revendications

1. Procédé de préparation de 1,3-propanediol par hydratation de l'acroléine en présence d'une résine échangeuse cationique acide en formant le 3-hydroxypropionaldéhyde, de l'acroléine et de l'eau étant mis à réagir en proportion pondérale de 1 : 2 à 1 : 10 entre 30 et 120°C et sous une pression comprise entre 1 et 10 bars, séparation de l'échangeur ionique et, s'il y a en a, de l'acroléine qui n'a pas réagi du mélange réactionnel et ensuite hydrogénation catalytique du 3-hydroxypropionaldéhyde dans les conditions connues en soi en phase liquide ou gazeuse en utilisant les catalyseurs usuels d'hydrogénation, caractérisé en ce qu'on utilise des résines échangeuses cationiques avec des groupes d'acides phosphoniques.

**2.** Procédé selon la revendication 1, caractérisé en ce qu'on utilise un échangeur cationique avec des groupes d'acides phosphoniques formant des chélates.

**3.** Procédé selon la revendication 1 ou 2, caractérisé en ce qu'on met en oeuvre à l'étape d'hydratation de l'acroléine et de l'eau en proportion pondérale de 1:3 à 1:6.

**4.** Procédé selon l'une ou plusieurs des revendications 1 à 3, caractérisé en ce qu'on réalise l'hydratation entre 50 et 90°C.

**5.** Procédé selon l'une ou plusieurs des revendications 1 à 4, caractérisé en ce qu'on réalise l'hydratation jusqu'à un taux de conversion de l'acroléine de 30 à 80 %, puis on sépare l'échangeur ionique et on élimine par distillation l'acroléine qui n'a pas réagi pour recyclage au stade d'hydratation à partir du mélange réactionnel aqueux.

**6.** Procédé selon l'une ou plusieurs des revendications 1 à 5, caractérisé en ce qu'on réalise l'hydratation dans un tube à écoulement, la conversion souhaitée en acroléine étant obtenue par un seul passage du mélange réactionnel dans le tube d'écoulement.

**7.** Procédé selon l'une ou plusieurs des revendications 1 à 6, caractérisé en ce qu'on réalise l'hydrogénation catalytique du 3-hydroxypropionaldéhyde en solution aqueuse à un pH compris entre 2,5 et 6,5 et à une température de 50 à 90°C.

**8.** Procédé selon l'une ou plusieurs des revendications 1 à 7, caractérisé en ce qu'on réalise l'hydrogénation du 3-hydroxypropionaldéhyde en présence de catalyseurs en suspension ou en lit fixe contenant du nickel.

## Claims

**1.** A process for the production of 1,3-propanediol by hydration of acrolein in the presence of an acidic cation exchanger resin to form 3-hydroxypropionaldehyde, acrolein and water being reacted in a ratio by weight of 1:2 to 1:20 at 30 to 120°C under a pressure of 1 to 20 bar, removal of the ion exchanger and the unreacted acrolein, if any, from the reaction mixture and subsequent catalytic hydrogenation of the 3-hydroxypropionaldehyde under conditions known per se in liquid or gas phase using typical hydrogenation catalysts, characterized in that cation exchanger resins containing phosphonic acid groups are used.

**2.** A process as claimed in claim 1, characterized in that cation exchangers containing chelate-forming aminophosphonic acid groups are used.

**3.** A process as claimed in claim 1 or 2, characterized in that acrolein and water in a ratio by weight of 1:3 to 1:6 are used in the hydration step.

**4.** A process as claimed in one or more of claims 1 to 3, characterized in that the hydration step is carried out at 50 to 90°C.

**5.** A process as claimed in one or more of claims 1 to 4, characterized in that the hydration step is continued to an acrolein conversion of 30 to 80%, the ion exchanger is subsequently removed and unreacted acrolein is then distilled off from the aqueous reaction mixture for recycling to the hydration step.

**6.** A process as claimed in one or more of claims 1 to 5, characterized in that the hydration step is carried out in a flow tube, the desired acrolein conversion being obtained by passing the reaction mixture once through the flow tube.

**7.** A process as claimed in one or more of claims 1 to 6, characterized in that the catalytic hydrogenation of the 3-hydroxypropionaldehyde is carried out in aqueous solution at a pH value of 2.5 to 6.5 and at a temperature of 50 to 90°C.

**8.** A process as claimed in one or more of claims 1 to 7, characterized in that 3-hydroxypropionaldehyde is hydrogenated in the presence of nickel-containing suspension or fixed-bed catalysts.